# EUROPEAN PATENT SPECIFICATION

(11) **EP 3 781 185 B1**
(45) Date of publication and mention of the grant of the patent: **09.08.2023**
(21) Application number: 19720497.7
(22) Date of filing: 17.04.2019
(51) Int. Cl.: A61K 35/745, A61K 35/747, A61P 25/34

(54) **METHOD FOR ALLEVIATING TOBACCO OR NICOTINE WITHDRAWAL SYMPTOMS**
VERFAHREN ZUR LINDERUNG VON TABAK- ODER NIKOTINENTZUGSERSCHEINUNGEN
PROCÉDÉ POUR SOULAGER LES SYMPTÔMES DE SEVRAGE DU TABAC OU DE LA NICOTINE

(30) Priority: 18.04.2018 EP 18290035
(43) Date of publication of application: 24.02.2021
(73) Proprietor: Danstar Ferment AG, 6300 Zug (CH)
(72) Inventor: FEUZ, Bérengère, 31620 Fronton (FR); GIRARD, Stéphanie-Anne, Québec H9S 4Y3 (CA)
(74) Representative: J A Kemp LLP
(86) International application number: PCT/EP2019/059996
(87) International publication number: WO 2019/202033

(56) References cited:
- EP-A1- 2 233 134
- WO-A1-2008/138571
- WO-A1-2016/124642
- WO-A2-2019/180263
- US-A1- 2006 269 535
- US-A1- 2013 295 069
- US-A1- 2014 079 676
- US-A1- 2015 335 688
- DIOP ET AL: "Probiotic food supplement reduces stress-induced gastrointestinal symptoms in volunteers: a double-blind, placebo-controlled, randomized trial", NUTRITION RESEARCH, ELSEVIER INC, XX, vol. 28, no. 1, 15 January 2008 (2008-01-15), pages 1-5, XP022420472, ISSN: 0271-5317, DOI: 10.1016/J.NUTRES.2007.10.001
- MICHA?L MESSAOUDI ET AL: "Assessment of psychotropic-like properties of a probiotic formulation ( Lactobacillus helveticus R0052 and Bifidobacterium longum R0175) in rats and human subjects", BRITISH JOURNAL OF NUTRITION, vol. 105, no. 05, 26 October 2010 (2010-10-26), pages 755-764, XP055192798, ISSN: 0007-1145, DOI: 10.1017/S0007114510004319
- AMY R ROMIJN ET AL: "A double-blind, randomized, placebo-controlled trial of Lactobacillus helveticus and Bifidobacterium longum for the symptoms of depression", AUSTRALIAN AND NEW ZEALAND JOURNAL OF PSYCHIATRY, vol. 51, no. 8, 1 August 2017 (2017-08-01) , pages 810-821, XP055487329, AU ISSN: 0004-8674, DOI: 10.1177/0004867416686694
- Douillard François P. ET AL: "Comparative Genomic and Functional Analysis of 100 Lactobacillus rhamnosus Strains and Their Comparison with Strain GG", PLoS Genetics, vol. 9, no. 8, 16 January 2013 (2013-01-16), page e1003683, XP055912801, DOI: 10.1371/journal.pgen.1003683 Retrieved from the Internet: URL:https://www.ncbi.nlm.nih.gov/pmc/artic les/PMC3744422/pdf/pgen.1003683.pdf>
- Anonymous: "Efficacy of 2 Probiotics During Nicotine Replacement Therapy on Withdrawal Symptoms Associated With Smoking Cessation - Full Text View - ClinicalTrials.gov", , 18 February 2016 (2016-02-18), XP055912800, Retrieved from the Internet: URL:https://clinicaltrials.gov/ct2/show/NC T02492022 [retrieved on 2022-04-14]
- Negin Noori ET AL: "Kefir protective effects against nicotine cessation-induced anxiety and cognition impairments in rats", Advanced Biomedical Research, vol. 3, no. 1, 1 January 2014 (2014-01-01) , page 251, XP055486745, ISSN: 2277-9175, DOI: 10.4103/2277-9175.146377

## Description

### TECHNOLOGICAL FIELD

The present disclosure relates to compositions for alleviating symptoms of tobacco or nicotine withdrawal and promoting smoking cessation. The compositions comprise at least one bacterial strain of a *Lactobacillus* species, at least one bacterial strain of a *Bifidobacterium* species and an acceptable carrier. The disclosure also relates to the composition for use in alleviating or suppressing the symptoms caused by withdrawal or partial withdrawal from the use of tobacco or of nicotine, methods of preparing such compositions, and the non-therapeutic use of at least one bacterial strain of a *Lactobacillus* species and at least one bacterial strain of a *Bifidobacterium* species for preparing such a composition.

### BACKGROUND

Tobacco smoke poses a serious threat to global health. In Canada, smoking is the leading cause of preventable disease, premature death and disability. Statistics Canada estimates that approximately 21 % of all deaths over the last decade are due to smoking and the World Health Organization (WHO) reports that tobacco alone is responsible for 6 million deaths annually world-wide.

Although the dangers of smoking are well known and documented, it is difficult for smokers to quit due to the addictive properties of nicotine. Indeed, nicotine interacts with nicotinic cholinergic receptors, facilitating neurotransmitter release and thereby mediating the complex actions of nicotine in tobacco users (Benowitz, 2009). Dopamine, glutamate, and gamma aminobutyric acid release are particularly important in the development of nicotine dependence. These chemical messengers are involved in reward behavior, reduction of anxiety, modulation of mood and appetite (Benowitz, 2008). It was also demonstrated that nicotine induces a decrease of the Hypothalamic-Pituitary-Adrenal (HPA) axis response to psychological stress, as well as a decrease in basal activity of the HPA axis (Rohleder et al., 2006).

Nicotine withdrawal is associated with a negative emotional state, including anxiety and the perception of increased stress, which may represent powerful stimuli to relapse to tobacco use. More particularly, quitting smoking is often related with stressful and uncomfortable nicotine withdrawal symptoms including: depression, anxiety, irritability, restlessness, hunger, lack of concentration, cravings for more nicotine and loss of sleep (Gritz et al., 1991; Hughes, 1992).

To help smokers quit, the pharmaceutical industry has developed different medicinal strategies as, for example nicotine replacement therapies (NRTs), to reduce withdrawal symptoms associated with cigarettes abstinence by replacing the nicotine from cigarettes. NRTs are commercially available under different forms: gum, transdermal patch, nasal spray, inhaler, oral spray, lozenge and sublingual tablet. It is well documented that NRTs are not very successful for long-term, quitting. A study found that 93% of the users relapse and return to smoking within six months (Hughes, 1992). Sides effects of NRT are generally mild and in the local area where the NRT is being used. The main side effects of NRT are: heart palpitations, chest pains, nausea and/or vomiting (oral products only), indigestion or gastrointestinal complaints (higher risk with oral products), insomnia (patch), mouth and throat soreness (oral products), mouth ulcers (oral products) and coughing (oral products).

Thus, there remains an acute need for safe and effective strategies and therapies that will alleviate or suppress the symptoms associated with tobacco or nicotine withdrawal in individuals attempting smoking cessation and increase the effectiveness of smoking cessation programs.

### SUMMARY OF THE DISCLOSURE

The present disclosure relates to a new method for alleviating or managing the adverse symptoms associated with tobacco or nicotine withdrawal and ease smoking cessation in individuals who wish to quit or decrease smoking tobacco or nicotine or the use of any tobacco product. The present disclosure relates to a method and composition for relieving and/or suppressing tobacco or nicotine craving and/or smoking withdrawal symptoms in individuals who wish to quit or decrease smoking tobacco or nicotine or the use of any tobacco product.

The present disclosure is based on the discovery that probiotic microorganisms are unexpectedly effective in palliating the effects of tobacco or nicotine withdrawals (or partial withdrawal), facilitate the cessation of tobacco or nicotine use and promote smoking cessation.

The invention is defined by the claims. Thus, the present invention provides a composition comprising at least one bacterial strain of a *Lactobacillus* species, at least one bacterial strain of a *Bifidobacterium* species and an acceptable carrier for use in alleviating or suppressing the symptoms caused by withdrawal or partial withdrawal from the use of tobacco or of nicotine, wherein said composition comprises: (a) at least one *L. rhamnosus* R0011 (LHS) strain and at least one *B. longum* R0175 (LHS) strain; or (b) at least one *L*. *rhamnosus* R0011 (LHS) strain and at least one *B. bifidum* R0071 (LHS) strain.

The present invention also provides a composition for alleviating or suppressing the symptoms caused by withdrawal or partial withdrawal from the use of tobacco or of nicotine, wherein said composition comprises: (a) at least one L. rhamnosus R0011 (LHS) strain and at least one B. longum R0175 (LHS) strain; or (b) at least one L. rhamnosus R0011 (LHS) strain and at least one B. bifidum R0071 (LHS) strain.

The present invention also provides a method of formulating a composition for alleviating or suppressing the symptoms caused by withdrawal or partial withdrawal from the use of tobacco or of nicotine, wherein said method comprises mixing: (a) at least one *L. rhamnosus* R0011 (LHS) strain and at least one *B. longum* R0175 (LHS) strain; or (b) at least one *L*. *rhamnosus* R0011 (LHS) strain and at least one *B. bifidum* R0071 (LHS) strain; with an acceptable carrier to prepare said composition.

The present invention further provides a non-therapeutic use of at least one bacterial strain of a *Lactobacillus* species and at least one bacterial strain of a *Bifidobacterium* species, for the preparation of a composition for alleviating or suppressing the symptoms caused by withdrawal or partial withdrawal from the use of tobacco or of nicotine, wherein said composition comprises: (a) at least one L. rhamnosus R0011 (LHS) strain and at least one B. longum R0175 (LHS) strain; or (b) at least one L. rhamnosus R0011 (LHS) strain and at least one B. bifidum R0071 (LHS) strain.

In the compositions, methods or uses of the present invention, the *Lactobacillus* species or strains and the *Bifidobacterium* species or strains may be present or mixed in a mutual weight ratio of between about 100:1 to about 1:100, between about 50:1 to about 1:50, between about 20:1 to about 1:20, between about 10:1 to about 1:10, between about 9:1 to about 1:9, between about 8:1 to about 1:8, between about 7:1 to about 1:7, between about 6:1 to about 1:6, between about 5:1 to about 1:5, between about 4:1 to about 1:4, between about 3:1 to about 1:3, between about 2:1 to about 1:2 or 1:1. For example, the bacterial species or strains may be present or mixed in a mutual weight ratio of about 4:1 of *Lactobacillus* species or strains to *Bifidobacterium* species or strains.

In the compositions, methods or uses of the present invention, said composition may comprise at least one *L. rhamnosus* R0011 (LHS) strain, at least one *B. longum* R0175 (LHS) strain, and at least *B. bifidum* R0071 (LHS) strain.

In the compositions, methods or uses of the present invention, said composition may be for use in combination with, or administered simultaneously or sequentially with, a nicotine replacement therapy, optionally wherein said nicotine replacement therapy is a gum, a transdermal patch, a nasal spray, an inhaler, an oral spray or a sublingual tablets/lozenges.

In the compositions, methods or uses of the present invention, the symptoms caused by withdrawal or partial withdrawal from the use of tobacco or of nicotine may comprise depression, irritability, anxiety, restlessness, hunger, lack of concentration, insomnia, nervous tremor, light-headedness or craving for tobacco or nicotine.

In the compositions, methods or uses of the present invention, the at least one bacterial strain of the *Lactobacillus* species, the at least one bacterial strain of the *Bifidobacterium* species, or a mixture thereof may be for use at a dosage of from about 1×10⁵ to 1×10¹² cfu total bacteria per dose, from about 1×10⁶ to 1×10¹¹ cfu total bacteria per dose, from about 1×10⁷ to 1×10¹⁰ cfu total bacteria per dose, or from about 1×10⁸ to 1×10¹⁰ cfu total bacteria per dose.

In the compositions, methods or uses of the present invention, said composition may be provided in the form of a freeze-dried power, a tablet, a capsule, a pill, a suspension, an emulsion, a liquid preparation, a gel or a syrup. In the compositions, methods or uses of the present invention, said composition may be provided in the form of a milk product, a yogurt, a curd, a cheese, a fermented milk, a milk powder, a milk based fermented product, an ice-cream, a fermented cereal based product, a milk based powder, a beverage, a dressing, a meat product, a spread, a filling, a frosting, a chocolate, a confectionery, a baked good, a sauces, a soup, a fruit juice, or a coffee whitener.

The present disclosure is further directed to a probiotic composition for use in a method for alleviating or suppressing the symptoms caused by withdrawal or partial withdrawal from the use of tobacco or of nicotine in an individual in need thereof comprising administering to the individual a therapeutically effective amount of a probiotic composition comprising at least one bacterial strain belonging to a *Lactobacillus* species and at least one bacterial strain of a *Bifidobacterium* species. The probiotic composition comprises (a) at least one *L. rhamnosus* R0011 (LHS) strain and at least one *B. longum* R0175 (LHS) strain, or (b) at least one *L*. *rhamnosus* R0011 (LHS) strain and at least one *B. bifidum* R0071 (LHS) strain. The probiotic composition may further comprise at least one microorganism strain belonging to a *Streptococcus* species, a *Enterococcus* species, a *Lactococcus* species, a *Bacillus* species or a *Saccharomyces* species. The at least one bacterial strain belonging to *Lactobacillus* species, the at least one bacterial strain belonging to *Bifidobacterium* species, or mixture thereof may be for use at a dosage of from about 1×10⁵ to 1×10¹² cfu total bacteria per dose, from about 1×10⁶ to 1×10¹¹ cfu total bacteria per dose, from about 1×10⁷ to 1×10¹⁰ cfu total bacteria per dose or from about 1×10⁸ to 1×10¹⁰ cfu total bacteria cfu per dose. The method may further comprise the administration of a nicotine replacement therapy. The nicotine replacement therapy may be a gum, a transdermal patch, a nasal spray, an inhaler, an oral spray or a sublingual tablets/lozenges. The nicotine replacement therapy may be a nicotine transdermal patch. The symptoms caused by withdrawal or partial withdrawal from the use of tobacco or of nicotine may comprise depression, irritability, anxiety, restlessness, hunger, lack of concentration, insomnia, nervous tremor, light-headedness or craving for tobacco or nicotine.

The symptoms caused by withdrawal or partial withdrawal from the use of tobacco or of nicotine may comprise depression, irritability, anxiety, restlessness, hunger, lack of concentration, insomnia, nervous tremor, light-headedness or craving for tobacco or nicotine.

The present disclosure also concerns a composition comprising at least one bacterial strain belonging to a *Lactobacillus* species and at least one bacterial strain of a *Bifidobacterium* species for alleviating or suppressing the symptoms caused by withdrawal or partial withdrawal from the use of tobacco or of nicotine in an individual in need thereof. The composition comprises (a) at least one *L. rhamnosus* R0011 (LHS) strain and at least one *B*. *longum* R0175 (LHS) strain, or (b) at least one *L. rhamnosus* R0011 (LHS) strain and at least one *B. bifidum* R0071 (LHS) strain. The probiotic composition may comprise at least one *L. rhamnosus* R0011 (LHS) strain, at least one *B. longum* R0175 (LHS) strain and at least one *B. bifidum* R0071 (LHS) strain.. The at least one bacterial strain belonging to *Lactobacillus* species, the at least one bacterial strain belonging to *Bifidobacterium* species, or mixture thereof may be for use at a dosage of from about 1×10⁵ to 1×10¹² cfu total bacteria per dose, from about 1×10⁶ to 1×10¹¹ cfu total bacteria per dose, from about 1×10⁷ to 1×10¹⁰ cfu total bacteria per dose or from about 1×10⁸ to 1×10¹⁰ cfu total bacteria cfu per dose. The composition of the present disclosure may further comprise the use of a nicotine replacement therapy. The nicotine replacement therapy may be a gum, a transdermal patch, a nasal spray, an inhaler, an oral spray or a sublingual tablets/lozenges. The nicotine replacement therapy may be a nicotine transdermal patch. The symptoms caused by withdrawal or partial withdrawal from the use of tobacco or of nicotine may comprise depression, irritability, anxiety, restlessness, hunger, lack of concentration, insomnia, nervous tremor, light-headedness or craving for tobacco or nicotine.

### BRIEF DESCRIPTION OF THE DRAWINGS

Having thus generally described the nature of the invention, reference will now be made to the accompanying drawings, in which:
Figure 1 illustrates *C. elegans* behavioral toxicity following 24 hours exposure to nicotine and tobacco extract. During the assay, the worms were also exposed to probiotic bacteria.
Figure 2 is a schematic representation of the percentage of participants who self-reported as abstinent but continued smoking, participants who reported smoking, and participants who were truly abstinent as determined by a carbon monoxide (CO) breath test (between week 2 and week 4).

### DETAILED DESCRIPTION

It has been surprisingly discovered that the use of the probiotic cultures of the genus *Bifidobacterium, Lactobacillus,* or a mixture thereof is beneficial in assisting to alleviate or suppress one or more withdrawal symptoms which afflict individuals who are trying to eliminate or decrease their use of tobacco or nicotine.

As is apparent from the examples below, it was found that the use of the probiotics *Bifidobacterium, Lactobacillus* or a mixture thereof proves to be especially useful for palliating tobacco or nicotine withdrawal (or partial withdrawal) symptoms or facilitating smoking cessation. The method of the present disclosure may be used in conjunction with a nicotine replacement therapy (NRT).

The common symptoms in individuals experiencing tobacco or nicotine withdrawal include, for example, depression, irritability, anxiety, restlessness, hunger, lack of concentration, insomnia, nervous tremor, light-headedness, and the craving for tobacco or nicotine. In addition to the difficulty related to cessation of smoking, common side effect that individuals experience when attempting to quit smoking is a substantial increase in appetite because they are craving food as a response to their anxiety. This increased hunger results in undesirable increases in body weight.

The term "promoting smoking cessation" as used herein refers to helping an individual to quit or reduce tobacco smoking or to quit or reduce use of tobacco products. The term "promoting smoking cessation" also refers to a reduction in a number of tobacco products smoked by an individual treated with the method of the present disclosure. Furthermore, the term "promoting smoking cessation" refers to alleviate, reduce, control, prevent, suppress or treat various symptoms associated with tobacco or nicotine withdrawal (including cravings for food) and/or to decrease craving for tobacco products or nicotine. The terms "alleviate, reduce or prevent" are intended to refer to any degree of reduction of the symptoms suffered by an individual.

The term "tobacco or nicotine withdrawal symptoms" as used herein refers to any physical or psychological reaction relating to breaking the habit of smoking tobacco or nicotine or using any tobacco product or decreasing the frequency or intensity of smoking tobacco or using any tobacco product.

The term "effective amount" as used herein is an amount of probiotics which is sufficient to produce the expected effect.

The term "probiotic microorganism" as used herein refers to a live microorganism which, when administered in adequate amounts, confers a health benefit to the host. A probiotic must fulfil several requirements related to lack of toxicity, viability, adhesion and beneficial effects.

As used herein, the term *"Bifidobacterium"* refers to members of the genus *Bifidobacterium.* These bacteria are Gram-positive anaerobic bacteria that are one of the major strains of bacteria present in the gastrointestinal flora. Specific examples of *Bifidobacterium* species include *B. bifidum, B. animalis* subsp. *lactis, B. breve, B. longum, or B. longum* subsp. *infantis.* The *Bifidobacterium* strains used in the invention are set out in claim 1. Exemplary species and strains of *Bifidobacterium* include the following well-known strains: *B. bifidum* HA-132 (sold by Lallemand Health Solutions ("LHS")), *B. bifidum* R0071 (LHS), *B. breve* HA-129 (LHS), *B. breve* R0070 (LHS), *B. infantis* HA-116 (LHS), *B. infantis* R0033 (LHS), *B*. *lactis* HA-194 (LHS), *B. longum* HA-135 (LHS), *B. longum* R0175 (LHS), *B. animalis* subsp. *lactis* R0421 (LHS). The *Bifidobacterium* used in the invention may be *B. longum* R0175 (LHS). *B. longum* R0175 (LHS) is also referred to as *B. longum* susp. *longum* R0175, *B*. *longum* susp. *longum* Rosell^{®}-175, *B. longum* Rosell^{®}-175, and Rosell^{®}-175. These terms are used interchangeably herein. Alternatively, the *Bifidobacterium* used in the invention is *B. bifidum* R0071 (also referred to as *B. bifidium* Rosell^{®}-71 or Rosell^{®}-71). Other bacterial strains having a name including an *"R"* code, e.g. *"R0123",* may be commercially known with respect to the name "*Rosell*^{®}", e.g. "*Rosell*^{®}*-123*". It is intended that the genus include species that have been reclassified (e.g., due to changes in the speciation of organisms as the result of genetic and other investigations) or renamed for marketing and/or other purposes.

As used herein, the term *"Lactobacillus"* refers to members of the genus *Lactobacillus,* in the family Lactobacillaceae. These bacteria are Gram-positive facultatively anaerobic bacteria that represent a major part of the bacterial group often referred to as "lactic acid bacteria." *Lactobacillus* species include *L. acidophilus, L. brevis, L. bulgaricus, L. casei, L. crispatus, L. delbrueckii, L. fermentum, L. gasseri, L. helveticus, L. lactis, L. plantarum, L. reuteri, L. rhamnosus, L. salivarius or L. paracasei.* The *Lactobacillus* strain used in the invention is set out in claim 1. Exemplary species and strains of *Lactobacillus* include the following well-known strains: *L. acidophilus* HA-122 (LHS), *L. acidophilus* R0418 (LHS), *L. brevis* HA-112 (LHS), *L. casei* HA-108 (LHS), *L. casei* R0215 (LHS), *L. delbrueckii bulgaricus* HA-137 (LHS), *L. fermentum* HA-179 (LHS), *L. helveticus* HA-128 (LHS), *L. helveticus* HA-501 (LHS), *L. helveticus* R0052 (LHS), *L. helveticus* Lafti L10 R0419 (LHS), *L. paracasei* HA-196 (LHS), *L. paracasei* HA-274 (LHS), *L. paracasei* Lafti L26 R0422 (LHS), *L. plantarum* R0403 (LHS), *L. plantarum* R0202 (LHS), *L. plantarum* R1012 (LHS), *L. reuteri* HA-188 (LHS), *L*. *rhamnosus* HA-114 (LHS), *L. rhamnosus* HA-500 (LHS), *L. rhamnosus* R0011 (LHS), *L*. *rhamnosus* R0049 (LHS), *L. rhamnosus* R0343 (LHS), *L. rhamnosus* R1039 (LHS), *L*. *salivarius* HA-118 (LHS), *L. salivarius* R0078 (LHS), *L. bulgaricus* R0440 (LHS) or *L. lactis* R1087 (LHS). The *Lactobacillus* used in the invention is *L. rhamnosus* R0011 (LHS) (interchangeably referred to as *L. rhamnosus* Rosell^{®}-11 or Rosell^{®}-11). It is intended that the genus include species that have been reclassified (e.g., due to changes in the speciation of organisms as the result of genetic and other investigations) or renamed for marketing and/or other purposes.

One probiotic microorganism (e.g. a strain) of the present disclosure may be used alone or in combination with or in conjunction with one or more different probiotic microorganisms. As used herein, the term "in combination with or in conjunction with" means together, substantially simultaneously or sequentially. A bacterium (e.g. a strain) of the genus *Lactobacillus* or *Bifidobacterium* can be used in combination or in conjunction with at least one bacterial strain from within the same species and not bacteria from any other species. At least a bacterium (e.g. a strain) of the genus *Lactobacillus* or *Bifidobacterium* can be used in combination or in conjunction with more than one species from within the same genus and not bacteria from any other genus. At least a bacterium (e.g. a strain) of the genus *Bifidobacterium* may be used in combination or in conjunction with at least a bacterium (e.g. a strain) of the genus *Lactobacillus. L. rhamnosus* can be provided in combination with *B*. *bifidum, B. longum* or a mixture thereof. The *L. rhamnosus* is *L. rhamnosus* R0011 (LHS). The *B. bifidum* is *B. bifidum* R0071. The *B. longum* is *B. longum* subsp. *longum* R0175 (LHS). It will be appreciated that the probiotics of the present disclosure may be combined with other suitable microorganisms (e.g. strains of probiotics bacteria) or yeasts. Examples of suitable microorganisms are yeast such as *Saccharomyces,* and bacteria such as the genera *Propionibacterium, Streptococcus, Enterococcus, Lactococcus, Bacillus, Pediococcus, Oenococcus* or combination thereof. *Saccharomyces* species include, without limitation, *S*. *cerevisiae* or *S*. *cerevisiae var. boulardii.* Specific examples of suitable probiotic microorganisms are: *Enterococcus faecium* HA-127 (LHS), *Enterococcus faecium* R0026 (LHS), *Lactococcus lactis* subsp. *lactis* HA-136 (LHS), *Lactococcus lactis* R1058 (LHS), *Streptococcus thermophilus* HA-110 (LHS), *Streptococcus thermophilus* R0083 (LHS), *Bacillus subtilis* HA-124 (LHS), *Bacillus subtilis* R0179 (LHS), *Propionibacterium freudenreichii* HA-273 (LHS), *Propionibacterium shermanii* HA-182 (LHS) or *Pediococcus acidilacticii* R1001 (LHS). Multiple strains of the same bacteria species may be utilized in combination.

The effective amount of colony forming units ("cfu") for each strain in the composition will be determined by the skilled in the art and will depend upon the final formulation. The term "colony forming unit" is defined herein as number of bacterial cells as revealed by microbiological counts on agar plates. For instance, the total probiotic may be provided in an amount of from about 10⁵ to 10¹² colony forming units (cfu) per dose, from about 10⁶ to 10¹¹ cfu per dose, from about 10⁷ to 10¹⁰ cfu per dose or from about 10⁸ to 10¹⁰ cfu per dose. The probiotic may be provided in an amount greater than about 1.0 × 10⁹ cfu total probiotic per dose. The individual may be administered greater than 5.0 × 10⁹ cfu total probiotic per dose. However, it is not intended that the present disclosure be limited to a specific dosage as it is contemplated that dosages of total probiotic will vary depending upon a number of factors such as the identity and number of individual probiotic strains employed, the subject being treated, the nature of the symptoms suffered by the subject that is to be treated, the general health of the subject, and the form in which the composition is administered.

When a bacterium of the genus *Bifidobacterium* or a bacterium of the genus *Lactobacillus* is used in combination with one different probiotic microorganism (e.g. a different strain), the bacteria may be present in any ratio capable of achieving the desired effects of the disclosure described herein. Typically, the bacterial species or strains constituting the probiotic mixture are present in a mutual weight ratio of between about 100:1 to about 1:100, between about 50:1 to about 1:50, between about 20:1 to about 1:20, between about 10:1 to about 1:10, between about 9:1 to about 1:9, between about 8:1 to about 1:8, between about 7:1 to about 1:7, between about 6:1 to about 1:6, between about 5:1 to about 1:5, between about 4:1 to about 1:4, between about 3:1 to about 1:3, between about 2:1 to about 1:2 or 1:1.

For example, when a bacterium of the genus *Bifidobacterium* is used in combination with a bacterium of the genus *Lactobacillus,* the bacteria may be present in any ratio capable of achieving the desired effects of the disclosure described herein. Typically, the *Lactobacillus* to *Bifidobacterium* are present in a mutual weight ratio of between about 100:1 to about 1:100, between about 50:1 to about 1:50, between about 20:1 to about 1:20, between about 10:1 to about 1:10, between about 9:1 to about 1:9, between about 8:1 to about 1:8, between about 7:1 to about 1:7, between about 6:1 to about 1:6, between about 5:1 to about 1:5, between about 4:1 to about 1:4, between about 3:1 to about 1:3, between about 2:1 to about 1:2 or 1:1.

More particularly, when *B. longum* is used in combination with *L. rhamnosus,* the bacteria species or strains constituting the mixture are present in a *Lactobacillus* to *Bifidobacterium* mutual weight ratio of between about 100:1 to about 1:100, between about 50:1 to about1:50, between about 20:1 to about 1:20, between about 10:1 to about 1:10, between about 9:1 to about 1:9, between about 8:1 to about 1:8, between about 7:1 to about 1:7, between about 6:1 to about 1:6, between about 5:1 to about 1:5, between about 4:1 to about 1:4, between about 3:1 to about 1:3, between about 2:1 to about 1:2 or 1:1.

In another example, when *B. bifidum* is used in combination with *L. rhamnosus,* the bacteria species or strains constituting the mixture are present in a *Lactobacillus* to *Bifidobacterium* mutual weight ratio of between about 100:1 to about 1:100, between about 50:1 to about 1:50, between about 20:1 to about 1:20, between about 10:1 to about 1:10, between about 9:1 to about 1:9, between about 8:1 to about 1:8, between about 7:1 to about 1:7, between about 6:1 to about 1:6, between about 5:1 to about 1:5, between about 4:1 to about 1:4, between about 3:1 to about 1:3, between about 2:1 to about 1:2 or 1:1.

When at least three or more probiotic strains are used in combination, the weight ratio of individual strains constituting the mixture may be from about 100:1 to about :1:100, between about 50:1 to about 1:50, between about 20:1 to about 1:20, between about 10:1 to about 1:10, between about 9:1 to about 1:9, between about 8:1 to about 1:8, between about 7:1 to about 1:7, between about 6:1 to about 1:6, between about 5:1 to about 1:5, between about 4:1 to about 1:4, between about 3:1 to about 1:3, between about 2:1 to about 1:2 or 1:1.

While it is possible to administer the probiotics of the present disclosure alone, they are typically administered on or in a support as part of a product, in particular as a component of a food product, a dietary supplement, medicament or a pharmaceutical formulation. These products typically contain additional components, acceptable excipients, carriers or adequate additives well known to those skilled in the art. The term "acceptable excipients and carriers" as used herein pertains to those that are compatible with the other ingredients in the formulation and biologically acceptable. The products may additionally contain one or more further active agents. The additional active agent or agents may be other probiotic bacteria or yeasts which are not antagonist to the strains forming the composition of the present disclosure. Depending on the formulation, the strains may be added as purified bacteria, as a bacterial culture, as part of a bacterial culture, as a bacterial culture which has been post-treated. Prebiotics could be also added. The food product, the dietary supplement or the pharmaceutical formulation may be prepared in any suitable form which does not negatively affect the bioavailability of the strains forming the composition and is within the scope of ordinary persons skilled in the art.

For example, the probiotic composition of the present disclosure can be formulated to be administered orally in the form of freeze-dried power, tablet, capsules, pills, suspension, lozenge, emulsion, liquid preparations, gel, syrup etc.

The probiotic composition of the present disclosure can be used as an ingredient in food products such as milk products, yogurt, curd, cheese (e.g. quark, cream, processed, soft and hard), fermented milk, milk powder, milk based fermented product, ice-cream, a fermented cereal based product, milk based powder, a beverage, a dressing, meat products (e.g. liver paste, frankfurter and salami sausages or meat spreads), spreads, fillings, frostings, chocolate, confectionery (e.g. caramel, candy, fondants or toffee), baked goods (cakes, pastries), sauces and soups, fruit juices or coffee whiteners.

The probiotic microorganisms are produced by cultivating the microorganisms in a suitable medium and under suitable conditions as known in the art. The probiotic microorganisms can be cultivated alone to form a pure culture, or as a mixed culture together with other microorganisms, or by cultivating probiotic microorganisms of different types separately and then combining them in the desired proportions. After cultivation until a predetermined CFU/g concentration is reached, the cell suspension is recovered and used as such or treated in the desired manner, for instance, by concentrating, spray drying, lyophilization, flatbed oven drying or freeze-drying, to be further employed in the preparation of composition and can be blend with a carrier medium. Sometimes the probiotic preparation is subjected to an immobilisation or encapsulation process in order to improve the shelf life. Several techniques for immobilisation or encapsulation of bacteria are known in the art.

The probiotic strains used in the present disclosure are in the form of viable cells. However, the probiotic strains of the present disclosure can also be in the form of non-viable cells such as killed cultures or compositions containing beneficial factors produced by the probiotics. This could include thermally killed micro-organisms or micro-organisms killed by exposure to altered pH, sonication, radiation or subjection to pressure. With non-viable cells product preparation is simpler, cells may be incorporated easily into commercial products and storage requirements are much less limited than viable cells.

In accordance with the present disclosure, probiotic compositions will normally be administered so that a symptom-ameliorating effective daily dose is received by the subject. The daily dose may be given in divided doses as necessary, the precise amount of the compound or agent received and the route of administration depending on the general health of the subject being treated according to principles known in the art. A typical dosage regime is once, twice or three daily.

The probiotic composition of the present disclosure can be used in conjunction with a nicotine replacement therapy such as, but not limited to, gum, nicotine transdermal patches, nasal spray, inhaler, oral spray or sublingual tablets/lozenges. The nicotine replacement therapy may be nicotine transdermal patches.

The word "comprising" in the claims may be replaced by "consisting essentially of" or with "consisting of," according to standard practice in patent law.

The present invention will be more readily understood by referring to the following examples which are given to illustrate the invention rather than to limit its scope.

### EXAMPLES

EXAMPLE 1: Effect of *B. bifidum, B. longum* and *L. rhamnosus* on *Caenorhabditis elegans* model submitted to nicotine and tobacco extract.

### Objective:

The objective of this study was to evaluate the effect of *B. bifidum, B. longum, L. rhamnosus* and combination thereof on the behavior of *C. elegans* submitted to nicotine and tobacco extract.

### Cultures of B. bifidum, B. longum and L. rhamnosus:

*L. rhamnosus* R0011 strain was grown anaerobically in MRS broth at 37 °C overnight. *B*. *longum* R0175 was grown anaerobically in MRS broth supplemented with 0.05% cysteine at 37 °C overnight.

Overnight cultures of strains R0011 and R0175 along with 1 g of lyophilized powder of *B*. *bifidum* R0071 were washed once with M9 buffer and resuspended in 10 ml of M9 buffer after centrifugation for 10 minutes at 4000rpm. The number of viable bacterial cells used in the experiment was analyzed on the flow cytometer and the fluorescent signal from the bacteria was detected and a count per unit volume was determined and adjusted at a concentration of 2×10⁸ cfu/ml.

The effect of each bacterial strain was tested alone and in combination. *L. rhamnosus* R0011 and *B. longum* R0175 were blended in a ratio of 80:20. *L. rhamnosus* R0011 and *B*. *bifidum* R0071 were blended in a ratio of 80:20. A third blend was tested and combined *L*. *rhamnosus* R0011, *B. longum* R0175 and *B. bifidum* R0071 in a ratio of 80:10:10.

### Synchronization of C. elegans:

Experiments were carried out with the wild type strain N2 of *C. elegans*. Worms were synchronized by isolating eggs from gravid adults, collecting the eggs in M9 buffer, hatching the eggs overnight in NGM plates and sterilely isolating L1-stage worms with M9 buffer. The L1-stage worms were transferred to NGM (Nematode Growth Medium) plates inoculated with *Escherichia coli* strain OP50 and incubated for four days at 15°C to reach adulthood.

### Preparation of the nicotine and tobacco extract:

Nicotine ditartrate dihydrate 100 mM (Acros) was diluted in M9 buffer to a final concentration of 6.2 mM. Similarly, a natural tobacco extract (ABT#6079, Advanced Biotech) was diluted in M9 buffer to a final concentration of 6.2 mM.

### Assay:

Adult worms were washed in M9 buffer and 250 µl of worm suspension was transferred to 24-well culture plates with or without nicotine (250 µl if present) or tobacco extract (250 µl if present) and with or without probiotic suspension (500 µl if present). The probiotic suspension was added first to the wells followed by the nicotine or the tobacco extract. At least 40 worms were present per well. The plates were incubated at 20°C for 24 hours. After incubation period, worm suspension in each well was washed three times in M9 buffer (centrifugation of four minutes at 1000 rpm). The worms were transferred in new 24-well culture plates containing 1 ml of agar 1% in each well. The worms were allowed to equilibrate for 45 minutes, and then the thrashing (swimming) was recorded using a Leica EC3 camera. The average thrashings per second were computed and then plotted for all of the probiotic strains and combinations of strains.

### Results and conclusion:

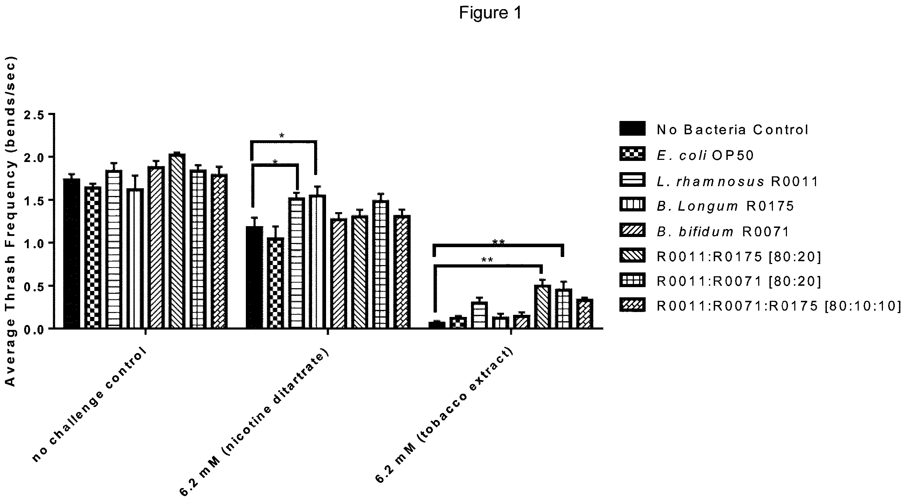

As shown in Figure 1, millimolar exposures of nicotine induced a reversible paralysis to *C. elegans.* This is represented in the drop in performance of control worms incubated with no bacteria and challenged with pure nicotine compared to no challenge control. Co-incubation with *L. rhamnosus* R0011, *B. longum* R0175, combinations of *L. rhamnosus* R0011 with *B*. *longum* R0175, *L. rhamnosus* R0011 with *B. bifidum* R0071 and the combination of the three probiotic strains seemed to prevent the decrease in swimming speed induced by the exposure to nicotine ditartrate dihydrate (Acros). This could possibly be achieved by preventing the over-neuroexcitiation by nicotine and/or accelerating/aiding the recovery post exposure.

In addition to nicotine, the tobacco extract contain other constituents that were highly toxic to the worms and induced a greater paralysis/death (or decreased the thrashing and body bend) as compared to the assay performed with nicotine (Figure 1). The results showed that *L. rhamnosus* R0011 alone, combinations of *L. rhamnosus* R0011 with *B. longum* R0175, *L*. *rhamnosus* R0011 with *B. bifidum* R0071 and the combination of the three probiotic strains attenuated the toxic effect of tobacco extract in *C. elegans.*

EXAMPLE 2: Clinical trial assessing the effectiveness of two probiotic products during nicotine replacement therapy for managing withdrawal symptoms associated with smoking cessation

### Objective:

The objective of this clinical study was to evaluate the efficacy or potential of the probiotics *B. bifidum, B. longum* subsp. *longum* and *L. rhamnosus* during nicotine replacement therapy for managing withdrawal symptoms associated with smoking cessation.

### Study design:

The study was a double-blind, prospective, randomized, placebo controlled trial. The total study duration was 16 weeks. The randomization was kept blinded to the patient and the investigators.

### Study population:

Seventy-five healthy participants between 18 and 65 years of age, moderate or heavily smokers were selected for the trial. Moderate or heavily smokers are defined by a Fagerström score ≥ 5 and who smoke more than 10 cigarettes per day. Participants were also selected for their willingness to stop smoking, corresponding to a score of 6 or 7 to the Motivation To Stop Scale (MTSS) (Kotz et al., 2013). The MTSS is a one-item questionnaire with 7 possible choices, from 1 (lowest level of motivation to stop smoking) to level 7 (highest level of motivation to stop smoking). Moreover, the participants had to discontinue consumption of probiotic supplements and food containing added probiotics or and/or prebiotics (e.g. yoghourts, with live, active cultures or supplements).

The trial has one placebo control group (Group 1) and two experimental treatment groups (Group 2 and Group 3). Subjects are assigned to one of the following treatment groups:
Group 1: placebo
Group 2: probiotic product 1
Group 3: probiotic product 2

### Test products:

Probiotic product 1 (*Lactobacillus rhamnosus* R0011 and *Bifidobacterium bifidum* R0071):
The probiotic product 1 under investigation is a combination of *Lactobacillus rhamnosus* R0011 and *Bifidobacterium bifidum* R0071 under the form of freeze-dried bacteria standardized with potato starch and magnesium stearate. The concentration of total probiotic is 5 × 10⁹ CFU per capsule after 2 years at 25°C. The ratio of both bacteria is *L. rhamnosus* R0011: *B*. *bifidum* R0071 is 4:1 after 2 years at 25°C.

Probiotic product 2 (*Lactobacillus rhamnosus* R0011 and *Bifidobacterium longum* subsp. *longum* R0175):
The probiotic product 2 under investigation is a combination of *L. rhamnosus* R0011 and *B*. *longum* subsp. *longum* R0175, under the form of freeze-dried bacteria standardized with potato starch and magnesium stearate. The concentration of total probiotic is 5 × 10⁹ CFU per capsule after 2 years at 25°C. The ratio of both bacteria is *L. rhamnosus* R0011: *B. longum* subsp. *longum* R0175 4:1 after 2 years at 25°C.

The placebo contains potato starch and magnesium stearate. Probiotics in both combinations appear as a white powder with small beige specks inside a transparent capsule.

### Treatment administration and procedure:

Participants were randomized in three groups receiving either one of the probiotic product or the placebo. It has been shown that withdrawal symptoms are important during the first days of tobacco abstinence. Therefore, at day 0 participants received the investigation product (IP) for 2 weeks before the quit day and beginning of NRT (at week 2), to increase the potential to detect effects on withdrawal symptoms. Nicotine patches (active ingredient: nicotine and non-medicinal ingredients: acrylate adhesive, aluminized polyester, silicone adhesive) were used as NRT. The probiotic supplementation was provided for 14 weeks, i.e. from D0 to week 14 while the nicotine patches were provided for 10 weeks, i.e. from week 2 to week 12.

The three groups of participants were followed-up during 16 weeks. All participants had a study visit at Day -2 (Screening Visit), at D0, at week 2 (D14), at week 4 (D28), at week 12 (Day 84), at week 14 (Day 98) and a follow-up visit at week 16 (Day 112). One phone call was performed at week 8.

Participants were requested to complete a daily diary to assess compliance to IP and NRT, rate the number of cigarettes smoked, assess withdrawal symptoms (using MPSS), bowel movements and stool consistency, daily throughout the study. Evolution of anxiety and depression symptoms, nicotine addiction, body weight and food cravings were also be scored during the study.

### Study assessments:

During all the period of the study, participants were completing a daily report to rate their withdrawal symptoms using the mood and physical symptoms scale (MPSS) (Prapavessis et al. 2007; Javors et al. 2011; Coleman et al. 2012).

The MPSS is a 12-item scale which can be divided in 3 subscales (West, 2012):
- MPSS(M), assessing mood: depression, anxiety, irritability, restlessness, hunger, lack of concentration and sleep (insomnia).
- MPSS(C), assessing craving: urgency in terms of number of craving per day and strength of the craving.
- MPSS(P), assessing physical symptoms: sores in the mouth, constipation, cough/sore throat.

The MPSS(M) score, corresponding to the sum of the 7 items for the MPSS(M) subscale, was compared between groups, daily between week 2 and 4 (first 2 weeks of tobacco abstinence) and week 12 and 14 (first 2 weeks after the end of NRT), and weekly for the other weeks. For weekly comparisons, an average of daily scores was done for the respective weeks. The score was also compared in time within groups, using the baseline ratings as covariates.

Furthermore, the following tests were conducted:
Hospital Anxiety and Depression Scale (HADS). The HADS is a widely used patient self-rated scale with 14 questions (7 "anxiety" and 7 "depression" questions) that ranges from 0-42. The higher the score, the more pronounced the symptom. A score of 7 indicates an absence of a disorder; a score ranging from 8 to 10 indicates a probable case of anxiety or depression; and, a score of ≥11 indicates a definite case of anxiety or depression. The HADS questionnaire will be administered at each visit.

The Fagerström Test for Nicotine Dependence (FTND). The FTND assess the evolution of nicotine addiction using a validated self-questionnaire. The FTND was used to assess dependence. No standard cut-off for nicotine dependence has been defined, but the following has been suggested: 1-2 = very low dependence; 3-4 = low dependence; 5 = medium dependence; 6-7 = high dependence; 8-10 = very high dependence (Fagerstrom et al., 1990).

Carbon monoxide levels in exhaled air. Carbon monoxide in exhaled air was measured in participants at each visit using a carbon monoxide monitor as an assessment of smoking cessation.

Salivary cotinine levels. Cotinine level monitoring can be used to provide an objective quantitative assessment of smoking status. Cotinine levels were determined in saliva samples by immunoassays. A participant was considered to be abstinent if the cotinine level measured in his saliva is less than 10 ng/ml (Prapavessis et al. 2007; Javors et al. 2011; Coleman et al. 2012).

Assessment of food cravings. Participants were asked to assess their food cravings weekly using the Questionnaire on Craving for Sweet or Rich Foods (QCSRF) (Toll et al., 2008).The QCSRF measures the intensity of craving for sweet or rich foods among smokers. The first question is about craving "at this moment" and the next 5 questions are related to the past week. All these six questions are rated from "none at all" to "more than ever" on a 7-point Likert scale. The 8 other questions assess current cravings using a 7-point Likert scale, from "strongly disagree" to "strongly agree". The evolution of the body weight was assed and measured at each visit.

Bristol Stool Scale. The Bristol Stool Scale is seven-point scale measuring stool consistency in adults with functional gastrointestinal disorders. In order to assess the effect of probiotics and smoking cessation on daily bowel movement frequency, participants were asked daily to rate the consistency of each of their bowel movement using the Bristol Stool Scale.

Microbiome Analysis and Persistence of Strains. Stool samples were collected at week 0 and at the end of week 14. Changes in the microbiome composition of participants as well as persistence of the probiotic strains were determined by genotypic methods.

### Results:

The scores between Group 1 (placebo - 1629), Group 2 (probiotic product 1 - 4682) and Group 3 (probiotic product 2 - 8213) were recorded between weeks 1-2 (Pre-Baseline - prior to smoking cessation and beginning of NRT) until weeks 3-4 (NRT Therapy - during smoking cessation). The analysis of data of the Mood and Physical Symptoms Scale (MPSS(M)) showed that the participants taking the probiotic product 2 (*Lactobacillus rhamnosus* R0011 and *Bifidobacterium longum* subsp. *longum* R0175) had the lowest increase in withdrawal symptoms from pre-baseline to NRT therapy when compared to the placebo group (Table 1). More particularly, the use of probiotic product 2 resulted in a smaller overall difference in the withdrawal symptoms amongst all three groups. Although these results did not reach statistical significance (p=0.4478), they demonstrated that the probiotic product 2 have a greater efficacy in mitigating withdrawal symptoms. In conclusion, the probiotic product 2 reduced depression, anxiety, and stress and improved positive mood in a higher proportion.

**Table 1: Changes in mean MPSS (M) Score for Smoking-Abstinent Participants by Treatment Group (F = 0.8752; p = 0.4478)**

| **Group** | **Pre-Baseline (mean ± SD)** | **NRT Therapy (mean ± SD)** | **Correlation** | **Difference (Δ ± SE)** | **p-Value** |
|---|---|---|---|---|---|
| **Placebo** | 10.66 ± 1.88 | 12.31 ± 2.05 | 0.99 | 1.65 ± 0.13 | 0.0010 |
| **Probiotic product 1** | 8.29 ± 0.96 | 10.99 ± 3.03 | 0.58 | 2.70 ± 0.98 | 0.0327 |
| **Probiotic product 2** | 11.66 ± 4.59 | 12.68 ± 4.21 | 0.88 | 1.03 ± 0.74 | 0.2030 |

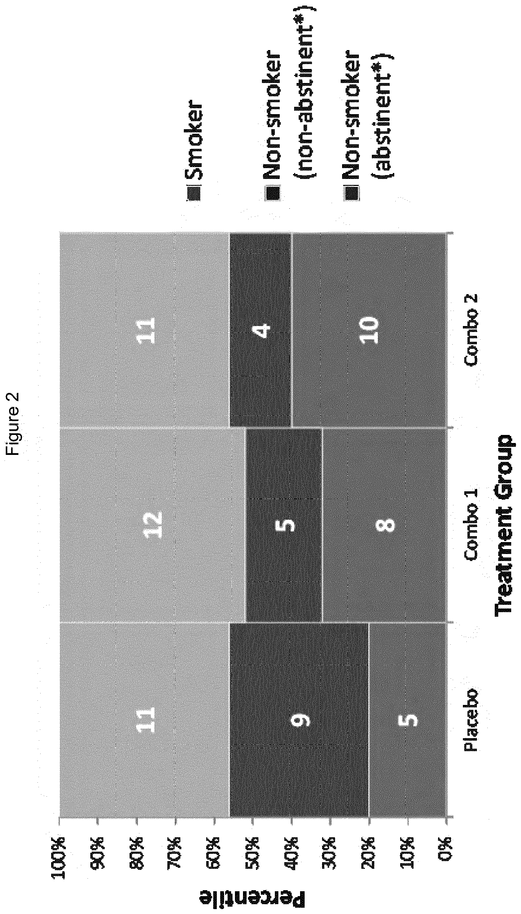

As illustrated in Figure 2, both probiotic products improved smoking abstinence in participants between week 2 and week 4. Figure 2 depicts the number and percentage of participants who self-reported as abstinent but continued smoking, participants who reported smoking, and participants who were truly abstinent as determined by a carbon monoxide (CO) breath test.

Furthermore, a CO breath test was conducted on the remaining seven participants in each of the three groups at end of the study (week 16) in order to determine the number of truly abstinent participants. The results shown in Table 2 indicated that both probiotic products had a greater proportion of truly abstinent participants compared to the placebo group. Probiotic product 2 was again the most effective in maintaining smoking abstinence.

Thus, it is contemplated that these probiotics (product 1: *Lactobacillus rhamnosus* R0011 and *Bifidobacterium bifidum* R0071 an product 2: *Lactobacillus rhamnosus* R0011 and *Bifidobacterium longum* subsp. *longum* R0175) will find use in the management of withdrawal symptoms associated with smoking cessation and in maintaining smoking abstinence.

**Table 2: Smoking Cessation Status at week 16**

| | | | **Probiotic product 1** | **Probiotic product 2** | **Placebo (N=24)** | **Total (N=75)** |
|---|---|---|---|---|---|---|
| | | | **R0011** + **R0071 (N=26)** | **R0011 + R0175 (N=25)** | | |
| Visit 6 (Week 16) - End of study | CO concentration in exhaled air (ppm) | n | 7 | 7 | 7 | 21 |
| | | Non-smoker (0-10ppm) | 4 (57.1 %) | 7 (100.0%) | 3 (42.9%) | 14 (66.7%) |
| | | Smoker (> 10ppm) | 3 (42.9%) | 0 (0.0%) | 4 (57.1 %) | 7 (33.3%) |

The analysis of data of the Mood and Physical Symptoms Scale (MPSS(C)) assessing craving, shown in Table 3, demonstrated that the probiotic products 1 and 2 were effective in reducing tobacco cravings or the urge to smoke.

**Table 3: Changes in mean MPSS (C) Score for Smoking-Abstinent Participants by Treatment Group (F = 0.2504; p = 0.7809)**

| **Group** | **Pre-Baseline (mean ± SD)** | **NRT Therapy (mean ± SD)** | **Correlation** | **Difference (Δ ± SD)** | **p-Value** |
|---|---|---|---|---|---|
| **Placebo** | 5.71 ± 0.71 | 5.05 ± 2.01 | +0.62 | -0.66 ± 1.72 | 0.4403 |
| **Probiotic product 1** | 5.87 ± 0.87 | 5.10 ± 1.67 | +0.84 | -0.77 ± 1.06 | 0.0396 |
| **Probiotic product 2** | 5.33 ± 0.59 | 4.61 ± 0.75 | +0.52 | -0.72 ± 0.66 | 0.0119 |

### REFERENCES

Benowitz, N.L. Clinical pharmacology of nicotine: implications for understanding, preventing, and treating tobacco addiction. Clinical pharmacology and therapeutics. 2008;83(4):531-41.
Benowitz, N.L. Pharmacology of nicotine: addiction, smoking-induced disease, and therapeutics.Annu Rev Pharmacol Toxicol. 2009;49:57-71.
Coleman T, Cooper S, Thornton JG, Grainge MJ, Watts K, Britton J, et al. A randomized trial of nicotine-replacement therapy patches in pregnancy. The New England journal of medicine. 2012;366(9):808-18.
Fagerstrom KO, Heatherton TF, Kozlowski LT. Nicotine addiction and its assessment. Ear, nose, & throat journal. 1990;69(11):763-5.
Gritz ER, Carr CR, Marcus AC. The tobacco withdrawal syndrome in unaided quitters. British journal of addiction. 1991;86(1):57-69.
Hughes JR. Tobacco withdrawal in self-quitters. Journal of consulting and clinical psychology. 1992;60(5):689-97.
Javors MA, Hatch JP, Lamb RJ. Sequential combination of self-report, breath carbon monoxide, and saliva cotinine to assess smoking status. Drug and alcohol dependence. 2011;113(2-3):242-4.
Kotz D, Brown J, West R. Predictive validity of the Motivation To Stop Scale (MTSS): a single-item measure of motivation to stop smoking. Drug and alcohol dependence. 2013;128(1-2):15-9.
Prapavessis H, Cameron L, Baldi JC, Robinson S, Borrie K, Harper T, et al. The effects of exercise and nicotine replacement therapy on smoking rates in women. Addictive behaviors. 2007;32(7):1416-32.
Rohleder N, Kirschbaum C. The hypothalamic-pituitary-adrenal (HPA) axis in habitual smokers. International journal of psychophysiology: official journal of the International Organization of Psychophysiology. 2006;59(3):236-43.
Toll BA, Katulak NA, Williams-Piehota P, O'Malley S. Validation of a scale for the assessment of food cravings among smokers. Appetite. 2008;50(1):25-32.
West R. Mood and Physical Symptoms Scale 2012. Available from the NCSCT (National Centre for Smoking Cessation and Training) website.

## Claims

1. A composition comprising at least one bacterial strain of a *Lactobacillus* species, at least one bacterial strain of a *Bifidobacterium* species and an acceptable carrier for use in alleviating or suppressing the symptoms caused by withdrawal or partial withdrawal from the use of tobacco or of nicotine, wherein said composition comprises:
(a) at least one *L. rhamnosus* R0011 (LHS) strain and at least one *B. longum* R0175 (LHS) strain; or
(b) at least one *L. rhamnosus* R0011 (LHS) strain and at least one *B. bifidum* R0071 (LHS) strain.

2. The composition for use according to claim 1, wherein the *Lactobacillus* species or strains and the *Bifidobacterium* species or strains are present in a mutual weight ratio of between about 100:1 to about 1:100, between about 50:1 to about 1:50, between about 20:1 to about 1:20, between about 10:1 to about 1:10, between about 9:1 to about 1:9, between about 8:1 to about 1:8, between about 7:1 to about 1:7, between about 6:1 to about 1:6, between about 5:1 to about 1:5, between about 4:1 to about 1:4, between about 3:1 to about 1:3, between about 2:1 to about 1:2 or 1:1, optionally wherein the bacterial species or strains are present in a mutual weight ratio of about 4:1 of *Lactobacillus* species or strains to *Bifidobacterium* species or strains.

3. The composition for use according to claim 1 or 2, wherein said composition comprises at least one *L. rhamnosus* R0011 (LHS) strain, at least one *B. longum* R0175 (LHS) strain, and at least *B. bifidum* R0071 (LHS) strain.

4. The composition for use according to any one of claims 1 to 3, wherein:
(a) said composition is to be used in combination with a nicotine replacement therapy, optionally wherein said nicotine replacement therapy is a gum, a transdermal patch, a nasal spray, an inhaler, an oral spray or a sublingual tablets/lozenges;
(b) the symptoms caused by withdrawal or partial withdrawal from the use of tobacco or of nicotine comprise depression, irritability, anxiety, restlessness, hunger, lack of concentration, insomnia, nervous tremor, light-headedness or craving for tobacco or nicotine; and/or
(c) the at least one bacterial strain of the *Lactobacillus* species, the at least one bacterial strain of the *Bifidobacterium* species, or a mixture thereof are for use at a dosage of from about 1×10⁵ to 1×10¹² cfu total bacteria per dose, from about 1×10⁶ to 1×10¹¹ cfu total bacteria per dose, from about 1×10⁷ to 1×10¹⁰ cfu total bacteria per dose, or from about 1×10⁸ to 1×10¹⁰ cfu total bacteria per dose.

5. The composition for use according to any one of claims 1 to 4, wherein said composition is provided in the form of:
(a) a freeze-dried power, a tablet, a capsule, a pill, a suspension, an emulsion, a liquid preparation, a gel or a syrup; or
(b) a milk product, a yogurt, a curd, a cheese, a fermented milk, a milk powder, a milk based fermented product, an ice-cream, a fermented cereal based product, a milk based powder, a beverage, a dressing, a meat product, a spread, a filling, a frosting, a chocolate, a confectionery, a baked good, a sauces, a soup, a fruit juice, or a coffee whitener.

6. A composition for alleviating or suppressing the symptoms caused by withdrawal or partial withdrawal from the use of tobacco or of nicotine, wherein said composition comprises:
(a) at least one *L. rhamnosus* R0011 (LHS) strain and at least one *B. longum* R0175 (LHS) strain; or
(b) at least one *L. rhamnosus* R0011 (LHS) strain and at least one *B. bifidum* R0071 (LHS) strain;
optionally wherein the composition is as defined in any one of claims 2 to 5.

7. A method of formulating a composition for alleviating or suppressing the symptoms caused by withdrawal or partial withdrawal from the use of tobacco or of nicotine, wherein said method comprises mixing:
(a) at least one *L. rhamnosus* R0011 (LHS) strain and at least one *B. longum* R0175 (LHS) strain; or
(b) at least one *L. rhamnosus* R0011 (LHS) strain and at least one *B. bifidum* R0071 (LHS) strain;
with an acceptable carrier to prepare said composition, optionally wherein said composition is as defined in any one of claims 2 to 5.

8. A non-therapeutic use of at least one bacterial strain of a *Lactobacillus* species and at least one bacterial strain of a *Bifidobacterium* species, for the preparation of a composition for alleviating or suppressing the symptoms caused by withdrawal or partial withdrawal from the use of tobacco or of nicotine, wherein said composition comprises:
(a) at least one *L. rhamnosus* R0011 (LHS) strain and at least one *B. longum* R0175 (LHS) strain; or
(b) at least one *L. rhamnosus* R0011 (LHS) strain and at least one *B. bifidum* R0071 (LHS) strain;
optionally wherein said composition is as defined in any one of claims 4 to 5.

## Patentansprüche

1. Zusammensetzung, die mindestens einen Bakterienstamm einer *Lactobacillus-*Spezies, mindestens einen Bakterienstamm einer *Bifidobacterium*-Spezies und einen akzeptablen Träger zur Verwendung bei der Linderung oder Unterdrückung von Symptomen umfasst, die durch den Entzug oder teilweisen Entzug vom Gebrauch von Tabak oder Nikotin verursacht werden, wobei die Zusammensetzung Folgendes umfasst:
(a) mindestens einen *L. rhamnosus* R0011 (LHS) Stamm und mindestens einen *B. longum* R0175 (LHS) Stamm; oder
(b) mindestens einen *L. rhamnosus* R0011 (LHS) Stamm und mindestens einen *B. bifidum* R0071 (LHS) Stamm.

2. Zusammensetzung zur Verwendung nach Anspruch 1, wobei die *Lactobacillus-*Spezies oder -Stämme und die *Bifidobacterium*-Spezies oder -Stämme in einem gegenseitigen Gewichtsverhältnis von zwischen etwa 100:1 bis etwa 1:100, zwischen etwa 50:1 bis etwa 1:50, zwischen etwa 20:1 bis etwa 1:20, zwischen etwa 10:1 bis etwa 1:10, zwischen etwa 9:1 bis etwa 1:9, zwischen etwa 8:1 bis etwa 1:8, zwischen etwa 7:1 bis etwa 1:7, zwischen etwa 6:1 bis etwa 1:6, zwischen etwa 5:1 bis etwa 1:5, zwischen etwa 4:1 bis etwa 1:4, zwischen etwa 3:1 bis etwa 1:3, zwischen etwa 2:1 bis etwa 1:2 oder 1:1 vorhanden sind, wobei die Bakterienarten oder -stämme optional in einem gegenseitigen Gewichtsverhältnis von etwa 4:1 von *Lactobacillus*-Spezies oder -Stämmen zu *Bifidobacterium*-Spezies oder -Stämmen vorhanden sind.

3. Zusammensetzung zur Verwendung nach Anspruch 1 oder 2, wobei die Zusammensetzung mindestens einen *L. rhamnosus* R0011 (LHS) Stamm, mindestens einen *B. longum* R0175 (LHS) Stamm und mindestens einen *B. bifidum* R0071 (LHS) Stamm umfasst.

4. Zusammensetzung zur Verwendung nach einem der Ansprüche 1 bis 3, wobei:
(a) die Zusammensetzung in Kombination mit einer Nikotinersatztherapie zu verwenden ist, wobei es sich bei der Nikotinersatztherapie optional um einen Kaugummi, ein transdermales Pflaster, ein Nasenspray, einen Inhalator, ein orales Spray oder eine sublinguale Tablette/Lutschtablette handelt;
(b) die durch den Entzug oder teilweisen Entzug von Tabak oder Nikotin verursachten Symptome Depressionen, Reizbarkeit, Angst, Unruhe, Hunger, Konzentrationsmangel, Schlaflosigkeit, nervöses Zittern, Schwindel oder Verlangen nach Tabak oder Nikotin umfassen; und/oder
(c) der mindestens eine Bakterienstamm der *Lactobacillus*-Spezies, der mindestens eine Bakterienstamm der *Bifidobacterium*-Spezies oder ein Gemisch davon zur Verwendung in einer Dosierung von etwa 1×10⁵ bis 1×10¹² cfu Gesamtbakterien pro Dosis, von etwa 1×10⁶ bis 1×10¹¹ cfu Gesamtbakterien pro Dosis, von etwa 1×10⁷ bis 1×10¹⁰ cfu Gesamtbakterien pro Dosis oder von etwa 1×10⁸ bis 1×10¹⁰ cfu Gesamtbakterien pro Dosis bestimmt ist.

5. Zusammensetzung zur Verwendung nach einem der Ansprüche 1 bis 4, wobei die Zusammensetzung in Form von Folgendem bereitgestellt wird:
(a) einer gefriergetrockneten Potenz, einer Tablette, einer Kapsel, einer Pille, einer Suspension, einer Emulsion, einer flüssigen Zubereitung, eines Gels oder eines Sirups; oder
(b) eines Milcherzeugnisses, eines Joghurts, eines Quarks, eines Käses, einer fermentierten Milch, eines Milchpulvers, eines fermentierten Erzeugnisses auf Milchbasis, eines Speiseeises, eines fermentierten Erzeugnisses auf Getreidebasis, eines Pulvers auf Milchbasis, eines Getränks, eines Dressings, eines Fleischerzeugnisses, eines Brotaufstrichs, einer Füllung, eines Zuckergusses, einer Schokolade, eines Konfekts, einer Backware, einer Soße, einer Suppe, eines Fruchtsafts oder eines Kaffeeweißers.

6. Zusammensetzung zur Linderung oder Unterdrückung von Symptomen, die durch den Entzug oder teilweisen Entzug von Tabak oder Nikotin verursacht werden, wobei die Zusammensetzung Folgendes umfasst:
(a) mindestens einen *L. rhamnosus* R0011 (LHS) Stamm und mindestens einen *B. longum* R0175 (LHS) Stamm; oder
(b) mindestens einen *L. rhamnosus* R0011 (LHS) Stamm und mindestens einen *B. bifidum* R0071 (LHS) Stamm;
wobei die Zusammensetzung optional nach einem der Ansprüche 2 bis 5 definiert ist.

7. Verfahren zur Formulierung einer Zusammensetzung zur Linderung oder Unterdrückung von Symptomen, die durch den Entzug oder teilweisen Entzug von Tabak oder Nikotin verursacht werden, wobei das Verfahren das Mischen von Folgendem umfasst:
(a) mindestens eines *L. rhamnosus* R0011 (LHS) Stamms und mindestens eines *B. longum* R0175 (LHS) Stamms; oder
(b) mindestens eines *L. rhamnosus* R0011 (LHS) Stamms und mindestens eines *B. bifidum* R0071 (LHS) Stamms;
mit einem annehmbaren Träger zur Herstellung der Zusammensetzung, wobei die Zusammensetzung optional nach einem der Ansprüche 2 bis 5 definiert ist.

8. Nicht-therapeutische Verwendung mindestens eines Bakterienstammes einer *Lactobacillus*-Spezies und mindestens eines Bakterienstammes einer *Bifidobacterium-*Spezies zur Herstellung einer Zusammensetzung zur Linderung oder Unterdrückung von Symptomen, die durch den Entzug oder teilweisen Entzug von Tabak oder Nikotin verursacht werden, wobei die Zusammensetzung Folgendes umfasst:
(a) mindestens einen *L. rhamnosus* R0011 (LHS) Stamm und mindestens einen *B. longum* R0175 (LHS) Stamm; oder
(b) mindestens einen *L. rhamnosus* R0011 (LHS) Stamm und mindestens einen *B. bifidum* R0071 (LHS) Stamm.
wobei die Zusammensetzung optional nach einem der Ansprüche 4 bis 5 definiert ist.

## Revendications

1. Composition comprenant au moins une souche bactérienne d'une espèce *Lactobacillus,* au moins une souche bactérienne d'une espèce *Bifidobacterium* et un support acceptable pour une utilisation dans le soulagement ou la suppression des symptômes provoqués par le sevrage ou le sevrage partiel de l'usage du tabac ou de la nicotine, dans laquelle ladite composition comprend :
(a) au moins une souche *L. rhamnosus* R0011 (LHS) et au moins une souche *B. longum* R0175 (LHS); ou
(b) au moins une souche *L. rhamnosus* R0011 (LHS) *et* au moins une souche *B. bifidum* R0071 (LHS).

2. Composition à utiliser selon la revendication 1, dans laquelle les espèces ou souches *Lactobacillus* et les espèces ou souches *Bifidobacterium* sont présentes dans un rapport pondéral mutuel compris entre environ 100:1 et environ 1:100, entre environ 50:1 et environ 1:50, entre environ 20:1 et environ 1:20, entre environ 10:1 et environ 1:10, entre environ 9:1 et environ 1:9, entre environ 8:1 et environ 1:8, entre environ 7:1 et environ 1:7, entre environ 6:1 et environ 1:6, entre environ 5:1 et environ 1:5, entre environ 4:1 et environ 1:4, entre environ 3:1 et environ 1:3, entre environ 2:1 et environ 1:2 ou 1:1, éventuellement dans laquelle les espèces ou souches bactériennes sont présentes dans un rapport pondéral mutuel d'environ 4:1 des espèces ou souches *Lactobacillus* par rapport aux espèces ou souches *Bifidobacterium .*

3. Composition à utiliser selon la revendication 1 ou 2, dans laquelle ladite composition comprend au moins une souche *L. rhamnosus* R0011 (LHS), au moins une souche *B. longum* R0175 (LHS) et au moins une souche *B. bifidum* R0071 (LHS).

4. Composition à utiliser selon l'une quelconque des revendications 1 à 3, dans laquelle :
(a) ladite composition doit être utilisée en combinaison avec une thérapie de remplacement de la nicotine, éventuellement dans laquelle ladite thérapie de remplacement de la nicotine est une gomme, un timbre transdermique, un vaporisateur nasal, un inhalateur, un vaporisateur oral ou des comprimés/pastilles sublinguaux ;
(b) les symptômes causés par le sevrage ou le sevrage partiel de l'usage du tabac ou de la nicotine comprennent la dépression, l'irritabilité, l'anxiété, l'agitation, la faim, le manque de concentration, l'insomnie, les tremblements nerveux, les étourdissements ou le besoin impérieux de tabac ou de nicotine ; et/ou
(c) l'au moins une souche bactérienne de l'espèce *Lactobacillus,* l'au moins une souche bactérienne de l'espèce *Bifidobacterium,* ou un mélange de celles-ci sont destinées à être utilisées à une dose d'environ 1 × 10⁵ à 1 × 10¹² cfu des bactéries totales par dose, d'environ 1×10⁶ à 1×10¹¹ cfu des bactéries totales par dose, d'environ 1×10⁷ à 1×10¹⁰ cfu des bactéries totales par dose, soit d'environ 1×10⁸ à 1×10¹⁰ cfu bactéries totales par dose.

5. Composition à utiliser selon l'une quelconque des revendications 1 à 4, dans laquelle ladite composition est fournie sous la forme de :
(a) une poudre lyophilisée, un comprimé, une gélule, une pilule, une suspension, une émulsion, une préparation liquide, un gel ou un sirop ; ou
(b) un produit laitier, un yaourt, un caillé, un fromage, un lait fermenté, un lait en poudre, un produit fermenté à base de lait, une crème glacée, un produit à base de céréales fermentées, une poudre à base de lait, une boisson, une vinaigrette, un produit de viande, une tartinade, une garniture, un glaçage, un chocolat, une confiserie, un produit de boulangerie, une sauce, une soupe, un jus de fruit ou un colorant à café.

6. Composition pour soulager ou éliminer les symptômes provoqués par le sevrage ou le sevrage partiel de l'usage du tabac ou de la nicotine, dans laquelle ladite composition comprend :
(a) au moins une souche *L. rhamnosus* R0011 (LHS) et au moins une souche *B. longum* R0175 (LHS) ; ou
(b) au moins une souche *L. rhamnosus* R0011 (LHS) et au moins une souche *B. bifidum* R0071 (LHS);
éventuellement dans laquelle la composition est telle que définie dans l'une quelconque des revendications 2 à 5.

7. Procédé de formulation d'une composition pour soulager ou éliminer les symptômes provoqués par le sevrage ou le sevrage partiel de l'usage du tabac ou de la nicotine, dans lequel ledit procédé comprend le mélange :
(a) d'au moins une souche *L. rhamnosus* R0011 (LHS) et d'au moins une souche *B. longum* R0175 (LHS) ; ou
(b) d'au moins une souche *L. rhamnosus* R0011 (LHS) et d'au moins une souche *B. bifidum* R0071 (LHS) ;
avec un support acceptable pour préparer ladite composition, éventuellement dans laquelle ladite composition est telle que définie dans l'une quelconque des revendications 2 à 5.

8. Utilisation non thérapeutique d'au moins une souche bactérienne d'une espèce *Lactobacillus* et d'au moins une souche bactérienne d'une espèce *Bifidobacterium,* pour la préparation d'une composition pour soulager ou éliminer les symptômes provoqués par le sevrage ou le sevrage partiel de l'usage du tabac ou de la nicotine, dans laquelle ladite composition comprend :
(a) au moins une souche *L. rhamnosus* R0011 (LHS) et au moins une souche *B. longum* R0175 (LHS) ; ou
(b) au moins une souche *L. rhamnosus* R0011 (LHS) et au moins une souche *B. bifidum* R0071 (LHS) ;
éventuellement dans laquelle ladite composition est telle que définie dans l'une quelconque des revendications 4 à 5.
